# EUROPEAN PATENT SPECIFICATION

(11) **EP 3 921 301 B1**
(45) Date of publication and mention of the grant of the patent: **05.04.2023**
(21) Application number: 20702663.4
(22) Date of filing: 06.02.2020
(51) Int. Cl.: C07C 315/00

(54) **PROCESS FOR PRODUCING 4,4'-DICHLORODIPHENYL SULFOXIDE**
VERFAHREN ZUR HERSTELLUNG VON 4,4'-DICHLORDIPHENYL-SULFOXID
PROCÉDÉ DE PRODUCTION DE 4,4'-DICHLORODIPHÉNYLE SULFOXIDE

(30) Priority: 08.02.2019 EP 19156189
(43) Date of publication of application: 15.12.2021
(73) Proprietor: BASF SE, 67056 Ludwigshafen am Rhein (DE)
(72) Inventor: THIEL, Indre, 67056 Ludwigshafen (DE); BEY, Oliver, 67056 Ludwigshafen (DE); SCHUETZ, Christian, 67056 Ludwigshafen (DE); BLEI, Stefan, 67056 Ludwigshafen (DE)
(74) Representative: BASF IP Association
(86) International application number: PCT/EP2020/052978
(87) International publication number: WO 2020/161232

(56) References cited:
- CN-A- 104 557 626
- SU-A1- 765 262
- XIAOPING SUN ET AL: "Iron(III) chloride (FeCl 3 )-catalyzed electrophilic aromatic substitution of chlorobenzene with thionyl chloride (SOCl 2 ) and the accompanying auto-redox in sulfur to give diaryl sulfides (Ar 2 S): Comparison to catalysis by aluminum chloride (AlCl 3 )", PHOSPHORUS, SULFUR AND SILICON AND THE RELATED ELEMENTS, vol. 192, no. 3, 7 October 2016 (2016-10-07), pages 376-380, XP055601069, US ISSN: 1042-6507, DOI: 10.1080/10426507.2016.1244206 cited in the application

## Description

The invention relates to a process for producing 4,4'-dichlorodiphenyl sulfoxide which also is called 1-chloro-4(4-chlorophenyl)sulfinyl benzene or bis(4-chlorophenyl)sulfoxide.

4,4'-dichlorodiphenyl sulfoxide (in the following DCDPSO) can be used as a precursor for producing 4,4-dichlorodiphenyl sulfone which is used for example as a monomer for preparing polymers such as polyarylene ethers like polysulfone, polyether sulfone, or polyphenylene sulfone or as an intermediate of pharmaceuticals, dyes and pesticides.

For the production of DCDPSO several processes are known. One process is a Friedel-Crafts reaction with thionyl chloride and chlorobenzene as starting materials in the presence of a catalyst, for example aluminum chloride. Generally, the reaction of thionyl chloride and chlorobenzene is disclosed as a first part in the production of 4,4'-dichlorodiphenyl sulfone, whereby an intermediate reaction product is obtained by the reaction of thionyl chloride and chlorobenzene which is hydrolyzed at an elevated temperature and thereafter oxidized to yield 4,4'-dichlorodiphenyl sulfone.

General processes for the production of sulfur containing diaryl compounds are disclosed for example in Sun, X. et al, "Investigations on the Lewis-acids-catalysed electrophilic aromatic substitution reactions of thionyl chloride and selenyl chloride, the substituent effect, and the reaction mechanisms", Journal of Chemical Research 2013, pages 736 to 744, Sun, X. et al, "Formation of diphenyl sulfoxide and diphenyl sulfide via the aluminum chloride-facilitated electrophilic aromatic substitution of benzene with thionyl chloride, and a novel reduction of sulfur(IV) to sulfur(II)", Phosphorus, Sulfur, and Silicon, 2010, Vol. 185, pages 2535-2542 and Sun, X. et al., "lron(ll) chloride (FeCl3)-catalyzed electrophilic aromatic substitution of chlorobenzene with thionyl chloride (SOCl2) and the accompanying auto-redox in sulfur to give diaryl sulfides (Ar2S): Comparison to catalysis by aluminum chloride (AlCl3)", Phosphorus, Sulfur, and Silicon, 2017, Vol. 192, No. 3, pages 376 to 380. In these papers different reaction conditions and catalysts are compared.

Friedel-Crafts acylation reactions of thionyl chloride and chlorobenzene in the presence of Lewis acid catalyst as part in the production of 4.4'-dichlorodiphenylsulfone are also disclosed for instance in CN-A 108047101, CN-A 102351756, CN-A 102351757, CN-A 102351758 or CN-A 104557626.

A two-stage process for producing 4,4'-dichlorodiphenyl sulfone where in the first stage DCDPSO is produced is disclosed in CN-B 104402780. For producing DCDPSO, a Friedel-Crafts reaction is described to be carried out at 20 to 30°C using thionyl chloride and chlorobenzene as raw material and anhydrous aluminum chloride as catalyst. The Friedel-Crafts reaction is followed by cooling, hydrolysis, heating and refluxing. It is further described that after reflux is finished the reaction mixture is cooled down and DCDPSO precipitates in form of white crystals which are filtered off. The DCDPSO then is oxidized to obtain 4,4'-dichlorodiphenyl sulfone. SU-A 765262 also discloses a two-stage process for producing 4,4'-dichlorodiphenyl sulfone where in the first stage DCDPSO is obtained by a Friedel-Crafts reaction using thionyl chloride and chlorobenzene in the presence of aluminum chloride at a temperature in the range from -10 to 50°C. According to the examples, the mixture obtained in the Friedel-Crafts reaction is poured into a 3% aqueous solution of hydrochloric acid and heated to completely dissolve the DCDPSO in the chlorobenzene which is added in excess. After separation into two phases, the organic phase is washed and then cooled to precipitate the DCDPSO. In one example the hydrochloric acid is obtained by trapping the hydrogen chloride evolved in the Friedel-Crafts reaction.

It is an object of the present invention to provide a reliable and energy-efficient process for producing 4,4'-dichlorodiphenyl sulfoxide with a reduced amount of impurities, particularly with a reduced amount of isomers like 2,4'-dichlorodiphenyl sulfoxide, 3,4'-dichlorodiphenylsulfoxide and 2,2'-dichlorodiphenyl sulfoxide.

This object is achieved by a process for producing DCDPSO comprising:
(a) reacting thionyl chloride, chlorobenzene and aluminum chloride in a molar ratio of thionyl chloride : chlorobenzene : aluminum chloride of 1 : (6 to 9) : (1 to 1.5) at a temperature in the range from 0 to below 20°C, forming an intermediate reaction product and hydrogen chloride,
(b) mixing aqueous hydrochloric acid and the intermediate reaction product at a temperature in the range from 70 to 110°C to obtain a crude reaction product comprising DCDPSO, wherein the aqueous hydrochloric acid has a concentration above 3wt% based on the total amount of hydrogen chloride and water in the aqueous hydrochloric acid,
(c) separating the crude reaction product into an organic phase comprising the DCDPSO and an aqueous phase,
(d) washing the organic phase with an extraction liquid.

It has been shown that by carrying out the reaction (a) at a temperature in the range from 0 to below 20°C a higher selectivity regards 4,4'-dichlorodiphenyl sulfoxide can be achieved.

By this process it is possible to achieve a final dry product comprising 4,4'-dichlorodiphenyl sulfoxide which contains less than 0.5 wt% isomers based on the total amount of all isomers of dichlorodiphenyl sulfoxide.

It is a further advantage of this process that the reaction product comprising DCDPSO is essentially free of aluminum chloride used as catalyst. "Essentially free" in this context means that, if at all detectable, there are only traces of aluminum chloride in the product obtained from the process, preferably, the amount of aluminum chloride is from 0 to 100 ppm, particularly less than 50 ppm.

To obtain DCDPSO, in the reaction (I) thionyl chloride, chlorobenzene and aluminum chloride are fed into a reactor in a molar ratio of thionyl chloride : chlorobenzene : aluminum chloride of 1 : (6 to 9) : (1 to 1.5), preferably in a molar ratio of thionyl chloride : chlorobenzene : aluminum chloride of 1 : (6 to 8) : (1 to 1.2) and particularly in a molar ratio of thionyl chloride : chlorobenzene : aluminum chloride of 1 : (6 to 7) : (1 to 1.1).

The reactor can be any reactor which allows mixing and reacting of the components fed into the reactor. A suitable reactor is for example a stirred tank reactor or jet loop reactor. If a stirred tank reactor is used, the stirrer preferably is an axially conveying stirrer, for example an oblique blade agitator. The reaction can be operated either continuously or batchwise. Preferably, the reaction is operated batchwise.

The thionyl chloride, chlorobenzene and aluminum chloride can be added simultaneously or successively. For reasons of ease of conduct of the reaction - in particular in case of batch reaction - preferably, aluminum chloride and chlorobenzene are fed firstly into the reactor and then the thionyl chloride is added to the aluminum chloride and chlorobenzene. In this case the aluminum chloride and chlorobenzene can be added simultaneously or one after the other. However, in each case it is preferred to mix the aluminum chloride and chlorobenzene before adding the thionyl chloride. Particularly preferably aluminum chloride and chlorobenzene are first fed into the reactor and the thionyl chloride is added to the aluminum chloride and chlorobenzene. During the reaction hydrogen chloride (HCl) - typically in gaseous form - is formed which is at least partially withdrawn from the reactor. The volumetric flow for adding the thionyl chloride typically depends on heat dissipation and flow rate of the gas withdrawn from the reactor.

The chlorobenzene which is added in excess into the reactor and, therefore, only partially converted during the chemical reaction, also serves as a solvent for the reaction products. In any step of the process in which a solvent is used, the solvent preferably is chlorobenzene. Due to the reaction conditions in the context of the present invention the person skilled in the art appreciates that the term "chlorobenzene" means monochlorobenzene which may contain traces of impurities.

The thionyl chloride and the chlorobenzene react in the presence of the aluminum chloride whereby an intermediate reaction product and hydrogen chloride form. The intermediate reaction product comprises 4,4'-dichlorodiphenyl sulfoxide-AlCl₃ adduct. The aluminum chloride generally can act as catalyst. The chemical reaction can be schematically represented by the following chemical reaction equation (1):

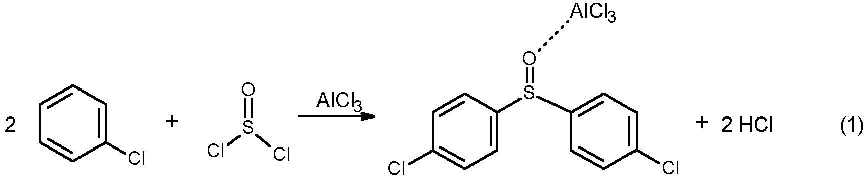

The reaction (a) is carried out at a temperature in the range from 0 to below 20°C, preferably at a temperature in the range from 3 to 15°C and particularly in the range from 5 to 12°C.

Thereby the reaction can be carried out at a constant or almost constant temperature. It is also possible to carry out the reaction at varying temperatures within the described ranges, for instance employing a temperature profile over the time of reaction or the reactor.

The reaction period generally depends on the amounts of reactants used and increases with increasing amounts of reactants. After addition of the thionyl chloride to the mixture of aluminum chloride and chlorobenzene is completed, the reaction preferably is continued for 10 to 120 min, more preferred from 20 to 50 min after the total amount of thionyl chloride is fed into the reactor.

Independently of whether the reaction is operated continuously or batchwise, the flow rate of the thionyl chloride is selected such that the heat generated by the reaction can be dissipated from the reactor by suitable cooling devices to keep the temperature in the reactor within a predefined range.

The hydrogen chloride (HCl) produced in the reaction typically is in gaseous form and at least partly removed from the reactor. While it can be put to other use in gaseous form, preferably, the hydrogen chloride removed from the reaction is mixed with water to produce aqueous hydrochloric acid.

After the reaction the intermediate reaction product is mixed with aqueous hydrochloric acid. For reasons of energy as well as production efficiency as well as sustainability, particularly preferably, the aqueous hydrochloric acid is produced from the hydrogen chloride removed from the reaction (a). By mixing the intermediate reaction product with the aqueous hydrochloric acid hydrolysis of the intermediate reaction product can take place. A crude reaction product comprising DCDPSO is obtained. The crude reaction product can also comprise aluminum chloride which is typically in hydrated form, usually as AlCl₃·6H₂O. The hydrolysis can be schematically represented by reaction equation (2):

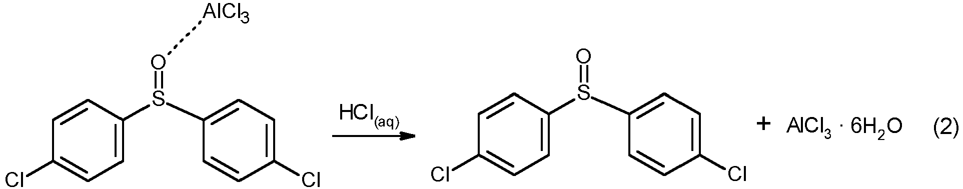

The concentration of the hydrochloric acid above 3 wt% improves the solubility of the aluminum chloride. Preferably, the aqueous hydrochloric acid used in the hydrolysis has a concentration in the range from above 3 to 12 wt%, more preferably in the range from 6 to 12 wt% and particularly preferably in the range from 10 to 12 wt%. All concentrations of hydrochloric acid in wt% above and in the following are based on the total amount of hydrogen chloride and water in the aqueous hydrochloric acid.

An advantage of a higher concentration, particularly of a concentration in the range from 10 to 12 wt%, is that the density of the aqueous phase increases and the aqueous phase thus forms the lower phase whereas the upper phase is the organic phase comprising the DCDPSO, in the following also termed as "organic phase". This allows an easier draining of the aqueous phase to obtain the organic phase. Further, the higher concentration allows a smaller amount of water for removing the aluminum chloride. A higher concentration of the aqueous hydrochloric acid further results in a quicker phase separation. It is a further advantage of the aqueous phase being the lower phase that for the easier draining of the aqueous phase the washing step (d) can be carried out in the same apparatus as the hydrolysis.

The temperature at which the hydrolysis is carried out is in the range from 70 to 110°C, preferably in the range from 80 to 100°C and particularly in the range from 80 to 90°C. The reaction period of the hydrolysis after all components for the hydrolysis are added preferably is in the range from 30 to 120 min, more preferred in the range from 30 to 60 min and particularly in the range from 30 to 45 min. This reaction period is in general sufficient for hydrolysis of the intermediate reaction product to obtain the DCDPSO. To facilitate the hydrolysis and to bring it as fast as possible to completion, the mixture can be agitated, preferably the mixture is stirred. After finishing the hydrolysis, the mixture separates into an aqueous phase comprising the AlCl₃ and an organic phase comprising DCDPSO solved in the excess chlorobenzene. In case the mixture is stirred, stirring is stopped to allow the mixture to separate.

The amount of aqueous hydrochloric acid used in (b) preferably is such that no aluminum chloride precipitates and that further two liquid phases are formed, the lower phase being the aqueous phase and the organic phase being the upper phase. To achieve this, the amount of aqueous hydrochloric acid used in (b) preferably is such that after the hydrolysis the weight ratio of aqueous to organic phase is in the range from 0.6 to 1.5 kg/kg, more preferably in the range from 0.7 to 1.0 kg/kg and particularly in the range from 0.8 to 1.0 kg/kg. A smaller amount of aqueous hydrochloric acid may result in precipitation of aluminum chloride. Particularly at higher concentrations of the aqueous hydrochloric acid a larger amount is necessary to avoid precipitation. Therefore, the concentration of the aqueous hydrochloric acid preferably is kept below 12 wt%.

The reaction of thionyl chloride, chlorobenzene and aluminum chloride and the mixing with aqueous hydrochloric acid and thus the hydrolysis can be carried out in the same reactor or in different reactors. Preferably, the reaction is carried out in a first reactor and the hydrolysis in a second reactor. If a first reactor and a second reactor are used, the first reactor corresponds to the reactor as described above. The second reactor also can be any reactor to perform a batchwise reaction and which allows agitating, preferably stirring of the components in the reactor. Therefore, the second reactor also preferably is a stirred tank reactor.

Either the one reactor, if the reaction and the hydrolysis are carried out in the same reactor, is or the preferably used first and second reactors are designed in such a way that the temperature can be set to adjust the temperature in the reactor. For this purpose, it is for example possible to provide a pipe inside the reactor through which a heating medium or a cooling medium can flow. Under the aspect of ease of reactor maintenance and/or uniformity of heating, preferably, the reactor comprises a double jacket through which the heating medium or cooling medium can flow. Besides the pipe inside the reactor or the double jacket the heating and/or cooling of the reactor(s) can be performed in each manner known to a skilled person.

If the reaction and the hydrolysis are carried out in different reactors, it is particularly preferred to heat the intermediate reaction product to a temperature which is above the solubility point of the intermediate reaction product in the solvent after the reaction is completed and prior to transporting the intermediate reaction product from the first reactor to the second reactor. Due to heating the intermediate reaction product before transporting and feeding into the second reactor, the intermediate reaction product dissolves and a liquid without solid components is transported. This has the advantage that fouling of the first reactor is avoided.

The solubility point denotes the temperature of the reaction mixture at which the intermediate reaction product is fully dissolved in the solvent. This temperature depends on the concentration of the intermediate reaction product in the solvent. The lower the concentration of DCDPSO in the organic phase, the lower the temperature at which the intermediate reaction product is fully dissolved in the solvent is.

If the reaction and the hydrolysis are carried out in the same reactor, the aqueous hydrochloric acid is fed into the reactor after the reaction is completed and after the intermediate reaction product is heated to the temperature of the hydrolysis. The flow rate of the aqueous hydrochloric acid preferably is set such that the temperature of the hydrolysis can be held in the specified range for the hydrolysis by tempering the reactor. If the reaction and the hydrolysis are carried out in different reactors, it is preferred to firstly feed the aqueous hydrochloric acid into the second reactor and to add the intermediate reaction product to the aqueous hydrochloric acid. In this case the flow rate of adding the intermediate reaction product into the second reactor is set such that the temperature in the second reactor is held within the specified temperature limits for the hydrolysis by tempering the second reactor.

To remove the aqueous hydrochloric acid and remainders of the aluminum chloride from the organic phase, the organic phase obtained in (c) is separated off and washed with an extraction liquid.

The phase separation following the hydrolysis can be carried out in the reactor in which the hydrolysis took place or in a separate vessel for phase separation. Under the aspect of less complexity, preferably the phase separation is carried out in the reactor in which the hydrolysis took place. After the phase separation is completed, the aqueous phase and the organic phase are removed separately from the vessel in which the phase separation took place, preferably the reactor in which the hydrolysis was performed. Using aqueous hydrochloric acid having a higher concentration for removing aluminum chloride, particularly aqueous hydrochloric acid having a concentration in the range from 10 to 12 wt% so that the density of the aqueous phase increases and the aqueous phase thus forms the lower phase, has the additional advantage that for the easier draining of the aqueous phase the washing of the organic phase can be carried out in the same apparatus as the hydrolysis.

After being separated off, the organic phase is fed into the washing step (d) to remove residual aluminum chloride and hydrochloric acid. The extraction liquid used for washing the organic phase preferably is water.

The washing preferably is carried out in a separate washing vessel. However, it is also possible to only remove the aqueous phase from the reactor in which the hydrolysis took place and carry out the washing step in the reactor in which the hydrolysis took place. If the washing is carried out in a separate washing vessel, any vessel in which an organic phase can be washed can be used. The washing vessel usually comprises means to intimately mix the organic phase with the extraction liquid. Preferably, the washing vessel is a stirred tank into which the organic phase and the extraction liquid are fed and then mixed.

If the phase separation is carried out in a vessel for phase separation, the washing either can be carried out in a washing vessel or, alternatively, in the vessel for phase separation. If phase separation and washing are carried out in the same vessel, it is necessary to provide means for mixing the organic phase with the extraction liquid after the aqueous phase which was separated from the organic phase is drained off.

The washing preferably is carried out at a temperature in the range from 70 to 110°C, more preferred in a range from 80 to 100°C and particularly in a range from 80 to 90°C. Particularly preferably the washing is carried out at the same temperature as the hydrolysis.

Generally, the amount of extraction liquid which preferably is water is sufficient to remove all or essentially all of the aluminum chloride from the organic phase. Under the aspect of waste control it is usually preferred to use as little extraction liquid as possible. The amount of water used for washing preferably is chosen in such a way that a weight ratio of aqueous to organic phase in the range from 0.3 to 1.2 kg/kg, more preferably in the range from 0.4 to 0.9 kg/kg and particularly in the range from 0.5 to 0.8 kg/kg is obtained. In terms of sustainability and avoidance of large waste water streams it is preferred to use as little water for the washing step as possible. It is particularly preferred to use such an amount of water that the entire aqueous phase from the washing step can be used to generate the aqueous hydrochloric acid in the concentration needed for hydrolysis.

After a predetermined washing period, mixing is stopped to allow the mixture to separate into an aqueous phase and an organic phase. The aqueous phase and the organic phase are removed from the washing vessel separately. The organic phase comprises the DCDPSO solved in the excess chlorobenzene as solvent. The predetermined washing period preferably is as short as possible to allow for short overall process times. At the same time, it needs sufficient time to allow for the removal of aluminum chloride.

The process may comprise one or more than one such washing cycles. Usually one washing cycle is sufficient.

The DCDPSO can be separated off the organic phase according to any process known to a skilled person. The organic phase for example can be cooled down to allow the DCDPSO to crystallize.

The aqueous phase removed from the washing preferably is used for producing the aqueous hydrochloric acid used for the hydrolysis. For this purpose, the water which is used for washing is separated off and mixed with the hydrogen chloride obtained in the reaction to obtain the aqueous hydrochloric acid. The mixing of the hydrogen chloride and the water can be performed for example in a washing column into which the gaseous hydrogen chloride and the water are fed. If such a washing column is used, preferably the hydrogen chloride and the water are fed in countercurrent. Besides a washing column all further vessels which allow absorbing the hydrogen chloride in water can be used. Thus, it is possible for example to feed the water into a vessel and to introduce the hydrogen chloride into the water. To introduce the hydrogen chloride into the water, for example a pipe can be used which immerges into the water. For distributing the hydrogen chloride in the water, it is possible to provide the end of the pipe immerging into the water with an immersion head having small holes through which the hydrogen chloride flows into the water. As an alternative, also a frit can be used for distributing the hydrogen chloride in the water.

Each process step described above can be carried out in only one apparatus or in more than one apparatus depending on the apparatus size and the amounts of compounds to be added. If more than one apparatus is used for a process step, the apparatus can be operated simultaneously or - particularly in a batchwise operated process - at different time. This allows for example to carry out a process step in one apparatus while at the same time another apparatus for the same process step is maintained, for example cleaned. Further, in that process steps where the contents of the apparatus remain for a certain time after all components are added, for example the reaction or the hydrolysis, it is possible after feeding all compounds in one apparatus to feed the components into a further apparatus while the process in the first apparatus still continues. However, it is also possible to add the components into all apparatus simultaneously and to carry out the process steps in the apparatus also simultaneously.

An illustrative embodiment of the invention is shown in the figure and explained in more detail in the following description.

In the drawing:

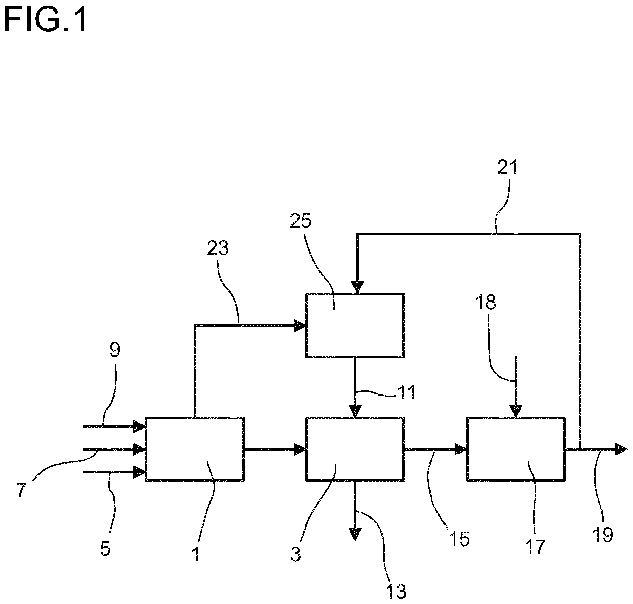

Figure 1 shows a schematic flow diagram of the process for producing DCDPSO.

An embodiment of the inventive process for producing DCDPSO is shown in the only figure.

The process for producing DCDPSO according to the embodiment as shown in figure 1 is carried out in a first reactor 1 and a second reactor 3. Chlorobenzene 5, thionyl chloride 7 as reactants and aluminum chloride 9 as catalyst are fed into the first reactor 1. The reactants and the catalyst can be fed simultaneously into the first reactor 1. However, preferably aluminum chloride 9 and chlorobenzene 5 are firstly fed into the first reactor 1 and mixed and the thionyl chloride 7 is then added to the mixture of aluminum chloride and chlorobenzene in a controlled way. In the first reactor 1 an intermediate reaction product is produced which is solved in excess chlorobenzene. The reaction in the first reactor is carried out at a temperature in the range from 0 to 15°C and ambient pressure. After the reaction is completed, the intermediate reaction product is withdrawn from the first reactor 1 and fed into the second reactor 3. Additionally, aqueous hydrochloric acid 11 with a concentration in the range from above 3 to 12 wt% is fed into the second reactor 3. In the second reactor 3 DCDPSO is produced from the intermediate product by hydrolysis.

The hydrolysis in the second reactor is performed at a temperature in the range from 70 to 110°C and at ambient pressure for 30 to 120 min. After finishing the hydrolysis, a phase separation into an aqueous phase and an organic phase takes place in the second reactor 3. The aqueous phase 13 containing aluminum chloride is removed from the process and the organic phase 15 comprising DCDPSO as product and chlorobenzene is fed into a washing device 17. In the washing device 17, the organic phase 15 comprising DCDPSO as product and chlorobenzene as solvent are mixed with water 18 to remove residual catalyst. The washing is performed at a temperature from 70 to 110°C and at ambient pressure. After the washing, the mixture separates into two phases, an aqueous phase comprising traces of chlorobenzene and aluminum chloride and an organic phase comprising DCDPSO as product and chlorobenzene as solvent. The organic phase is withdrawn from the process as product 19.

The organic phase withdrawn as product 19 then can be further treated to separate the DCDPSO from the chlorobenzene. One possibility to obtain DCDPSO is cooling the mixture whereby the DCDPSO precipitates and then can be filtered off.

Besides the intermediate reaction product hydrogen chloride accrues during the reaction in the first reactor 1. As the hydrogen chloride is gaseous, it easily can be withdrawn from the first reactor 1. The gaseous hydrogen chloride 23 preferably is fed into an absorbing device 25 as shown in the figure. In the absorbing device 25 aqueous hydrochloric acid is produced by absorbing the hydrogen chloride in water. This aqueous hydrochloric acid preferably is used for the hydrolysis in the second reactor 3 as shown in the figure.

The water for producing the aqueous hydrochloric acid in the absorption device 25 preferably is the aqueous phase 21 which emanates from the washing in the washing device 17. By using the aqueous phase 21 from the washing the total amount of fresh water can be reduced and thus a much smaller amount of wastewater accrues.

This wastewater is the aqueous phase from the hydrolysis in the second reactor 3. The wastewater can be disposed after cleaning.

### Examples

### Effect of the temperature in the first reaction

In all examples 5.5 mol aluminum chloride and 40 mol chlorobenzene were fed into a stirred tank reactor as first reactor. 5 mol thionyl chloride were added to the reaction mixture in 160 min. The reaction in the first reactor was carried out at different temperatures according to table 1. Hydrogen chloride produced in the reaction was withdrawn from the process. After finishing the addition of thionyl chloride, the reaction mixture was heated to 60°C.

After finishing the reaction in the first reactor, the resulting reaction mixture was fed into a second stirred tank reactor which contained 3400 g aqueous hydrochloric acid with a concentration of 11 wt%. The second stirred tank reactor was heated to a temperature of 90°C. After 30 min the reaction was finished, and the resulting reaction mixture was analyzed by GC analysis to determine the selectivity. The selectivity at the different reaction temperatures in the first reaction is also listed in table 1.

**Table 1: Selectivity towards 4,4'-dichlorodiphenyl sulfoxide at different temperatures of the first reaction**

| Example | 1 | 2 | 3 | 4 | 5 | 6 | 7 |
|---|---|---|---|---|---|---|---|
| Temperature [°C] | -3 | 0 | 6 | 10 | 20 | 30 | 40 |
| Selectivity [%] | 95,7 | 94,8 | 94,6 | 94,3 | 94 | 92,8 | 91,9 |

As can be seen from table 1, the selectivity decreases with increasing temperature, wherein there is only a small decrease in the range from 0 to 20°C. The highest selectivity can be observed at a temperature of -3°C. But since the reaction stops at temperatures below -3°C and thus a precise temperature control is necessary to avoid the temperature to fall below -3°C particularly in industrial scale processes the reaction is carried out at temperatures above -3°C.

### Effect of the concentration of the aqueous hydrochloric acid used in the hydrolysis

In all examples 5.5 mol aluminum chloride and 40 mol chlorobenzene were fed into a stirred tank reactor as first reactor. 5 mol thionyl chloride were added to the reaction mixture in 160 min. The reaction in the first reactor was carried out at 40°C. Hydrogen chloride produced in the reaction was withdrawn from the process. After finishing the addition of thionyl chloride the reaction mixture was heated to 60°C.

The intermediate reaction product produced in the first reaction was subjected to a hydrolysis in a second stirred tank reactor by adding aqueous hydrochloric acid. The amount and the concentration of the aqueous hydrochloric acid and the reaction time of the hydrolysis are listed in table 2. During hydrolysis, the mixture was stirred with a three-step cross-arm stirrer at 200 rpm. After hydrolysis, the mixing was stopped, and the mixture separated into an aqueous phase and an organic phase.

The aqueous phase was withdrawn, and the organic phase was washed with water while stirring. The washing duration and the amount of water also are listed in table 2. During washing the mixture was stirred with a three-step cross-arm stirrer at 100 rpm. After washing, stirring was finished and the mixture separated into an aqueous phase and an organic phase.

After phase separation the organic phase was subjected to a crystallization process. At 30°C the resulting suspension was filtered, and the filter cake washed with monochlorobenzene. Drying of the wet filter cake yielded the desired 4,4'-dichlorodiphenyl sulfoxide as a white crystalline solid. Example 1 does not form part of the claimed invention but serves comparative purposes.

**Table 2: Reaction conditions**

| | Example | 1 | 2 | 3 | 4 | 5 |
|---|---|---|---|---|---|---|
| Hydrolysis | Concentration HCl [wt%] | 3 | 6 | 10.7 | 16 | 12 |
| | Amount HCl [g] | 6990 | 3495 | 3495 | 2330 | 1747.5 |
| | reaction time hydrolysis [min] | 60 | 60 | 60 | 60 | 60 |
| | Phase separation time [s] | 130 | 140 | 140 | 165 | n.d. |
| Washing | Amount Water [g] | 4000 | 2000 | 2000 | 1333.3 | 1000 |
| | washing time [min] | 30 | 30 | 30 | 30 | 30 |
| | Phase separation time [s] | 30 | 40 | 30 | 30 | 50 |
| Yield | Final isolated yield [%] | 80.1 | 81.5 | 80.4 | 80.8 | 79.5 |

In example 5 it was not possible to separate the phases after hydrolysis because due to solubility issues the aluminum chloride precipitated.

In example 1 the aqueous phase was the upper phase after hydrolysis and after washing, in examples 2, 3 and 4 the aqueous phase was the bottom phase after hydrolysis and the upper phase after washing. In example 4, the aqueous phase after hydrolysis was very turbid.

## Claims

1. A process for producing 4,4'-dichlorodiphenyl sulfoxide comprising:
(a) reacting thionyl chloride, chlorobenzene and aluminum chloride in a molar ratio of thionyl chloride : chlorobenzene : aluminum chloride of 1 : (6 to 9) : (1 to 1.5) at a temperature in the range from 0 to below 20°C, forming an intermediate reaction product and hydrogen chloride,
(b) mixing aqueous hydrochloric acid and the intermediate reaction product at a temperature in the range from 70 to 110°C to obtain a crude reaction product comprising 4,4'-dichlorodiphenyl sulfoxide, wherein the aqueous hydrochloric acid has a concentration above 3 wt% based on the total amount of hydrogen chloride and water in the aqueous hydrochloric acid,
(c) separating the crude reaction product into an organic phase comprising the 4,4'-dichlorodiphenyl sulfoxide and an aqueous phase,
(d) washing the organic phase with an extraction liquid.

2. The process according to claim 1, wherein the hydrogen chloride obtained in (a) is mixed with water to obtain the aqueous hydrochloric acid which is added in (b).

3. The process according to claim 1 or 2 wherein the extraction liquid is water.

4. The process according to claim 3, wherein the water which is used for the washing the organic phase is separated off and mixed with the hydrogen chloride obtained in (a) to obtain the aqueous hydrochloric acid.

5. The process according to any of claims 1 to 4, wherein the washing is carried out at a temperature in the range from 70 to 110°C.

6. The process according to any of claims 1 to 5, wherein the aqueous hydrochloric acid has a concentration in the range from 3 to 12 wt%.

7. The process according to any of claims 1 to 6, wherein aluminum chloride and chlorobenzene are first fed into a reactor and the thionyl chloride is added to the aluminum chloride and chlorobenzene.

8. The process according to any of claims 1 to 7, wherein the amount of aqueous hydrochloric acid is such that the weight ratio of aqueous phase to organic phase of the crude reaction product is in the range from 0.6 to 1.5 kg/kg.

9. The process according to any of claims 1 to 8, wherein the amount of water used for washing in (d) is such that a weight ratio of aqueous phase to organic phase in the range from 0.3 to 1.2 kg/kg is obtained.

10. The process according to any of claims 1 to 9, wherein the reaction is carried out in a first reactor and the mixing of aqueous hydrochloric acid and the intermediate reaction product is carried out in a second reactor.

## Patentansprüche

1. Verfahren zur Herstellung von 4,4'-Dichlordiphenylsulfoxid, das Folgendes umfasst:
(a) Umsetzen von Thionylchlorid, Chlorbenzol und Aluminiumchlorid in einem Molverhältnis von Thionylchlorid : Chlorbenzol : Aluminiumchlorid von 1 : (6 bis 9) : (1 bis 1,5) bei einer Temperatur im Bereich 0 bis unter 20 °C unter Bildung von einem Reaktionszwischenprodukt und Chlorwasserstoff,
(b) Mischen von wässriger Salzsäure und dem Reaktionszwischenprodukt bei einer Temperatur im Bereich von 70 bis 110 °C zum Erhalt eines rohen Reaktionsprodukts, das 4,4'-Dichlordiphenylsulfoxid umfasst, wobei die wässrige Salzsäure eine Konzentration über 3 Gew.-%, bezogen auf die Gesamtmenge von Chlorwasserstoff und Wasser in der wässrigen Salzsäure, aufweist,
(c) Trennen des rohen Reaktionsprodukts in eine organische Phase, die das 4,4'-Dichlordiphenylsulfoxid umfasst, und eine wässrige Phase,
(d) Waschen der organischen Phase mit einer Extraktionsflüssigkeit.

2. Verfahren nach Anspruch 1, wobei der in (a) erhaltene Chlorwasserstoff mit Wasser gemischt wird, um die in (b) zugesetzte wässrige Salzsäure zu erhalten.

3. Verfahren nach Anspruch 1 oder 2, wobei es sich bei der Extraktionsflüssigkeit um Wasser handelt.

4. Verfahren nach Anspruch 3, wobei das zum Waschen der organischen Phase verwendete Wasser abgetrennt und mit dem in (a) erhaltenen Chlorwasserstoff gemischt wird, um die wässrige Salzsäure zu erhalten.

5. Verfahren nach einem der Ansprüche 1 bis 4, wobei das Waschen bei einer Temperatur im Bereich von 70 bis 110 °C durchgeführt wird.

6. Verfahren nach einem der Ansprüche 1 bis 5, wobei die wässrige Salzsäure eine Konzentration im Bereich von 3 bis 12 Gew.-% aufweist.

7. Verfahren nach einem der Ansprüche 1 bis 6, wobei Aluminiumchlorid und Chlorbenzol zuerst in einem Reaktor vorgelegt werden und das Thionylchlorid zu dem Aluminiumchlorid und dem Chlorbenzol gegeben wird.

8. Verfahren nach einem der Ansprüche 1 bis 7, wobei die Menge von wässriger Salzsäure so beschaffen ist, dass das Gewichtsverhältnis von wässriger Phase zu organischer Phase des rohen Reaktionsprodukts im Bereich von 0,6 bis 1,5 kg/kg liegt.

9. Verfahren nach einem der Ansprüche 1 bis 8, wobei die in (d) zum Waschen verwendete Wassermenge so beschaffen ist, dass ein Gewichtsverhältnis von wässriger Phase zu organischer Phase im Bereich von 0,3 bis 1,2 kg/kg erhalten wird.

10. Verfahren nach einem der Ansprüche 1 bis 9, wobei die Umsetzung in einem ersten Reaktor durchgeführt wird und das Mischen von wässriger Salzsäure und dem Reaktionszwischenprodukt in einem zweiten Reaktor durchgeführt wird.

## Revendications

1. Procédé pour la production de 4,4'-dichlorodiphénylsulfoxyde comprenant :
(a) la mise en réaction de chlorure de thionyle, de chlorobenzène et de chlorure d'aluminium en un rapport molaire de chlorure de thionyle : chlorobenzène : chlorure d'aluminium de 1 : (6 à 9) : (1 à 1,5) à une température dans la plage de 0 à en dessous de 20 °C, formant un produit de réaction intermédiaire et du chlorure d'hydrogène,
(b) le mélange d'acide chlorhydrique aqueux et du produit de réaction intermédiaire à une température dans la plage de 70 à 110 °C pour obtenir un produit de réaction brut comprenant du 4,4'-dichlorodiphénylsulfoxyde, l'acide chlorhydrique aqueux possédant une concentration supérieure à 3 % en poids sur la base de la quantité totale de chlorure d'hydrogène et d'eau dans l'acide chlorhydrique aqueux,
(c) séparation du produit de réaction brut en une phase organique comprenant le 4,4'-dichlorodiphénylsulfoxyde et une phase aqueuse,
(d) lavage de la phase organique avec le liquide d'extraction.

2. Procédé selon la revendication 1, le chlorure d'hydrogène obtenu en (a) étant mélangé avec de l'eau pour obtenir l'acide chlorhydrique aqueux qui est ajouté en (b).

3. Procédé selon la revendication 1 ou 2, le liquide d'extraction étant de l'eau.

4. Procédé selon la revendication 3, l'eau qui est utilisée pour le lavage de la phase organique étant séparée et mélangée avec le chlorure d'hydrogène obtenu en (a) pour obtenir l'acide chlorhydrique aqueux.

5. Procédé selon l'une quelconque des revendications 1 à 4, le lavage étant réalisé à une température dans la plage de 70 à 110 °C.

6. Procédé selon l'une quelconque des revendications 1 à 5, l'acide chlorhydrique aqueux possédant une concentration dans la plage de 3 à 12 % en poids.

7. Procédé selon l'une quelconque des revendications 1 à 6, le chlorure d'aluminium et le chlorobenzène étant d'abord alimentés dans un réacteur et le chlorure de thionyle étant ajouté au chlorure d'aluminium et au chlorobenzène.

8. Procédé selon l'une quelconque des revendications 1 à 7, la quantité d'acide chlorhydrique aqueux étant telle que le rapport en poids de phase aqueuse sur phase organique du produit de réaction brut soit dans la plage de 0,6 à 1,5 kg/kg.

9. Procédé selon l'une quelconque des revendications 1 à 8, la quantité d'eau utilisée pour le lavage en (d) étant telle qu'un rapport en poids de phase aqueuse sur phase organique dans la plage de 0,3 à 1,2 kg/kg soit obtenu.

10. Procédé selon l'une quelconque des revendications 1 à 9, la réaction étant réalisée dans un premier réacteur et le mélange d'acide chlorhydrique aqueux et du produit de réaction intermédiaire étant réalisé dans un deuxième réacteur.
